(19) **Europäisches Patentamt / European Patent Office / Office européen des brevets**

(11) **EP 4 576 008 A1**

(12) **EUROPEAN PATENT APPLICATION**

(43) Date of publication:
25.06.2025 Bulletin 2025/26

(21) Application number: 24220466.7

(22) Date of filing: 17.12.2024

(51) International Patent Classification (IPC):
*G06V 10/30* (2022.01)    *C12Q 1/6874* (2018.01)
*G01N 21/64* (2006.01)    *G06V 20/69* (2022.01)

(52) Cooperative Patent Classification (CPC):
(C-Sets available)
**G06V 10/30; C12Q 1/6874; G01N 21/6458;**
**G01N 21/6486; G06V 20/695;** G06V 2201/03;
G06V 2201/04        (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: 22.12.2023 CN 202311787884

(71) Applicant: **GeneMind Biosciences Company Limited**
**Shenzhen City, 518023 Guangdong (CN)**

(72) Inventors:
• **LI, Linsen**
**Shenzhen City, 518023 (CN)**
• **ZHOU, Yuan**
**Shenzhen City, 518023 (CN)**

(74) Representative: **Schiweck Weinzierl Koch**
**Patentanwälte Partnerschaft mbB**
**Ganghoferstraße 68 B**
**80339 München (DE)**

(54) **METHOD AND APPARATUS FOR DETERMINING INTER-CHANNEL CROSSTALK CORRECTION STRENGTH, AND MEDIUM**

(57) The present application discloses a method and apparatus for determining inter-channel crosstalk correction strength, and a medium. The method comprises: detecting multiple cycles of sequencing by synthesis based on a multi-channel microscopic imaging system, such that a first channel signal and a second channel signal are generated in each cycle, wherein a first nucleotide and a second nucleotide are nucleotides comprising different types of bases, and a part of the first channel signal is noise related to second channel crosstalk; detecting the first channel signal and the second channel signal in each cycle, wherein the first channel signal and the second channel signal both have intensity; and performing inter-channel crosstalk correction on the first channel signal intensity by using a predetermined coefficient and the second channel signal intensity to determine the crosstalk-corrected first channel signal intensity, wherein the predetermined coefficient is a fixed coefficient associated with multi-channel microscopic imaging. The present application can correct the inter-channel crosstalk, thereby improving the accuracy and efficiency of the signal correction and/or base calling method.

EP 4 576 008 A1

(52) Cooperative Patent Classification (CPC): (Cont.)

C-Sets
**C12Q 1/6874, C12Q 2565/601, C12Q 2563/107**

**Description**

## TECHNICAL FIELD

The present application relates to the technical field of data processing, and in particular, to a method and apparatus for determining inter-channel crosstalk correction strength, and a medium.

## BACKGROUND

Base calling is commonly applied in determining nucleic acid sequences. In particular, for platforms that use an optical imaging system to acquire images of signals of base extension multiple times and perform nucleic acid sequencing based on processing these images, accurate base calling based on these images and image signals is often difficult due to the effect of optical effects, spatial effects, and/or chemical reactions (such as chromatic aberration, crosstalk, and/or phasing) on image acquisition, localization, and/or signal intensity. The accuracy, efficiency, and the like of the existing signal correction methods and/or base calling methods need to be improved.

## SUMMARY

In view of this, the present application provides the following technical solutions:
Provided is a method for determining inter-channel crosstalk correction strength, which includes:

detecting multiple cycles of sequencing by synthesis based on a multi-channel microscopic imaging system, such that a first channel signal and a second channel signal are generated in each cycle, wherein the first channel signal is indicative of a signal of incorporation of a first nucleotide into a plurality of identical polynucleotide molecules in the cycle, the second channel signal is indicative of a signal of incorporation of a second nucleotide into the plurality of identical polynucleotide molecules in the cycle, the first nucleotide and the second nucleotide are nucleotides comprising different types of bases, and a part of the first channel signal is noise related to second channel crosstalk; detecting the first channel signal and the second channel signal in each cycle, wherein the first channel signal and the second channel signal both have intensity;
and performing inter-channel crosstalk correction on the first channel signal intensity by using a predetermined coefficient and the second channel signal intensity to determine the crosstalk-corrected first channel signal intensity, wherein the predetermined coefficient is a fixed coefficient associated with the multi-channel microscopic imaging system.

Provided is an apparatus for determining inter-channel crosstalk correction strength, which comprises:

a signal acquisition unit, configured for detecting multiple cycles of sequencing by synthesis based on a multi-channel microscopic imaging system, such that a first channel signal and a second channel signal are generated in each cycle, wherein the first channel signal is indicative of a signal of incorporation of a first nucleotide into a plurality of identical polynucleotide molecules in the cycle, the second channel signal is indicative of a signal of incorporation of a second nucleotide into the plurality of identical polynucleotide molecules in the cycle, the first nucleotide and the second nucleotide are nucleotides comprising different types of bases, and a part of the first channel signal is noise related to second channel crosstalk;
a signal detection unit, configured for detecting the first channel signal and the second channel signal in each cycle, wherein the first channel signal and the second channel signal both have intensity; and
a correction unit, configured for performing inter-channel crosstalk correction on the first channel signal intensity by using a predetermined coefficient and the second channel signal intensity to determine the crosstalk-corrected first channel signal intensity, wherein the predetermined coefficient is a fixed coefficient associated with the multi-channel microscopic imaging system.

Provided is a computer-readable storage medium having a program stored thereon, and the program can be executed by a processor to implement the method for determining the inter-channel crosstalk correction strength according to any one of the above.

It can be known from the above technical solutions that the present application discloses a method and apparatus for determining inter-channel crosstalk correction strength. The method includes: detecting multiple cycles of sequencing by synthesis based on a multi-channel microscopic imaging system, such that a first channel signal and a second channel signal are generated in each cycle, wherein the first channel signal is indicative of a signal of incorporation of a first nucleotide into a plurality of identical polynucleotide molecules in the cycle, the second channel signal is indicative of a signal of incorporation of a second nucleotide into the plurality of nucleotide molecules in the cycle, the first nucleotide and the second nucleotide are nucleotides comprising different types of bases, and a part of the first channel signal is noise related to second channel crosstalk; detecting the first channel signal and the second channel signal in each cycle, wherein the first channel signal and the second channel signal both have intensity; and performing inter-channel crosstalk correction on the first channel signal intensity by using a predetermined coefficient and the second channel signal intensity to determine the crosstalk-corrected first channel signal intensity, wherein the predetermined coefficient is a fixed coefficient associated with multi-channel microscopic imaging. The present application can correct the inter-channel crosstalk, thereby improving the accuracy and efficiency of the signal correction and/or base calling method.

## BRIEF DESCRIPTION OF THE DRAWINGS

In order to more clearly illustrate the technical solutions in the embodiments of the present application, the drawings required for use in the description of the embodiments will be briefly described below. It is obvious that the drawings in the description below are only embodiments of the present application, and other drawings can be derived from the provided drawings by those of ordinary skill in the art without making inventive efforts.

FIG. 1 is a schematic flow chart of a base calling method provided in an embodiment of the present application;
FIG. 2 shows crosstalk plots before one correction provided in an embodiment of the present application;
FIG. 3 shows crosstalk plots after one correction provided in an embodiment of the present application;
FIG. 4 is a schematic diagram of excitation spectra and emission spectra of different fluorophores provided in an embodiment of the present application;
FIG. 5 is a schematic diagram of spectra of emission light from different fluorophores entering a microscopic imaging system provided in an embodiment of the present application;
FIG. 6 is a schematic structural diagram of a base calling apparatus provided in an embodiment of the present application;
FIG. 7 shows crosstalk plots before another correction provided in an embodiment of the present application;
FIG. 8 shows crosstalk plots after another correction provided in an embodiment of the present application.

## DETAILED DESCRIPTION

The technical solutions in the embodiments of the present application will be described below clearly and comprehensively in conjunction with the drawings in the embodiments of the present application. It is evident that the described embodiments are part of the embodiments of the present application, but not all of them. Based on the embodiments of the present application, all other embodiments obtained by those of ordinary skill in the art without inventive efforts fall within the scope of protection of the present application.

In the embodiments of the present application, it should be understood that the terms "first" and "second" are used for illustrative purposes only, and should not be construed as indicating or implying the relative importance or implicitly indicating the number of indicated technical features; a feature defined by "first" and "second" may explicitly or implicitly include one or more of the features. Unless otherwise specified, "a group" or "a plurality of" refers to two or more. Unless otherwise specified, the singular forms "a", "an", "the", and the like include the singular or the plural (more than one) in number.

As used herein, unless otherwise specified, the term "comprise", "comprises", "comprising", "include", "includes", or "including" is open-ended and does not exclude the inclusion of other contents or situations that are consistent with the stated situations but not listed or exemplified herein.

As used herein, unless otherwise specified, a nucleotide refers to four natural nucleotides (e.g., dATP, dCTP, dGTP and dTTP, or ATP, CTP, GTP and UTP) or derivatives thereof, and is sometimes directly referred to as the base included (A, T/U, C, G). The reference to a nucleotide or base in a particular embodiment may be known to those of ordinary skills in the art in light of the context.

As used herein, unless otherwise specified, a polynucleotide molecule includes a single-stranded nucleic acid molecule, a double-stranded nucleic acid molecule, or a single-stranded double-stranded complex.

As used herein, the term "sequencing" refers to sequence determination, and is used interchangeably with "nucleotide sequencing" to refer to the determination of base order in nucleotide sequences, including sequencing by synthesis (SBS), including DNA sequencing and/or RNA sequencing, including long fragment sequencing and/or short fragment sequencing (the long fragment and short fragment are defined relatively; for example, nucleotide molecules longer than 1 Kb, 2 Kb, 5 Kb, or 10 Kb may be referred to as long fragments, and nucleotide molecules shorter than 1 Kb or 800 bp may be referred to as short fragments), and including double-end sequencing, single-end sequencing, and/or paired-end sequencing (the double-end sequencing or paired-end sequencing may refer to the reading of any two segments or portions of the same nucleotide molecule that are not completely overlapping).

In the present application, sequencing may be performed through a sequencing platform, and according to an embodiment of the present application, the sequencing platform that may be used to perform the above sequencing method may be selected from, but is not limited to, the Hiseq/Miseq/Nextseq/Novaseq sequencing platform (Illumina), the Ion Torrent platform (Thermo Fisher/Life Technologies), the BGISEQ and MGISEQ/DNBSEQ platforms (BGI) and single-molecule sequencing platforms. The sequencing method may be selected from single-end sequencing and double-end sequencing.

In the sequencing process based on sequencing by synthesis (SBS), the sequencing includes the process of linking or binding or incorporating nucleotides (including nucleotide analogs) to a template and acquiring the corresponding reaction signals under the catalysis of a polymerase. In particular, in the platform for SBS detection based on a microscopic imaging system, such as Miseq, Nextseq, and Novaseq platforms (ILLUMINA) and MGISEQ or DNBSEQ platform (MGI), modified nucleotides are used to controllably realize a polymerase chain reaction to achieve single base extension and acquire corresponding reaction signals, and the steps are repeated multiple times to achieve the determination of base order of a target nucleic acid molecule (also called a template). The repeated single base extension and acquisition of the corresponding signals are also commonly referred to as a sequencing cycle or one cycle of sequencing.

In the present application, the sequencing cycle or one cycle of sequencing represents a process of extending one base and determining the type of the extended base, i.e., in the sequencing cycle or one cycle of sequencing, a determination process for the type of the base at a given position on a template nucleotide molecule is completed, and this method is also called sequencing by synthesis (SBS). The determination of the order of multiple nucleotides/bases on the template is typically achieved by multiple cycles of sequencing.

The sequencing cycle or one cycle of sequencing may be defined as the completion of one base extension of four types of nucleotides/base, and in other words, as the determination of the base type at any given position on the template. For sequencing platforms that achieve sequencing based on SBS, the sequencing cycle or one cycle of sequencing includes the process of binding four types of nucleotides to the template at a time and acquiring the corresponding reaction signals. For SBS sequencing-based platforms, a reaction system includes a sequencing primer, a reaction substrate (nucleotides), a polymerase, and a template, and each cycle of sequencing includes the following processes: binding a predetermined sequence (the sequencing primer) to the template, and based on the base pairing principle and the rationale of polymerization reaction, controllably connecting the added reaction substrate (nucleotides) to the 3' end of the sequencing primer under the catalysis of the polymerase to achieve the pairing with the base at a corresponding position of the template. In general, the added reaction substrate nucleotides carry specific fluorophores, which are detected by the microscopic imaging system to determine the type of extended bases.

In the present application, the sequencing cycle or one cycle of sequencing may include one or more base extensions (repeats). For example, the extension and the acquisition of reaction signals may be performed by adding a mixture of all four types of nucleotides to the reaction system in one extension, and thus the determination of the extended bases may be completed by one extension. In addition, four types of nucleotides may also combined arbitrarily (e.g., combined in pairs) to determine the type of extended bases by at least one (e.g., two) extension and the acquisition of reaction signals.

It should be noted that, as will be understood by those skilled in the art, after each extension, a photograph may be taken by using the microscopic imaging system, and the fluorescence of different colors may be detected by using different channels. Thus, for each sequencing cycle or each cycle of sequencing, in order to determine the type of extended bases, at least one base image, or even a plurality of base images, needs to be collected, based on which the subsequent sequencing analysis may be performed.

Crosstalk, also called "laser-crosstalk" or "spectra-crosstalk", refers to the phenomenon in which the signal corresponding to one base diffuses into the signal of another base in one cycle of sequencing. For example, for a sequencing platform that uses fluorophores labeled differently to identify different bases, it may be detected that the signal of one fluorescent molecule diffuses from one channel into another channel in one cycle of sequencing if the emission spectra of two or more selected fluorophores overlap.

Phasing/prephasing, also called "phase imbalance", "dephasing", or "phase diversity", refers to the phenomenon of asynchrony of reactions among a plurality of identical polynucleotide molecules in a chemical reaction, including phasing/sequence lag and prephasing/sequence lead.

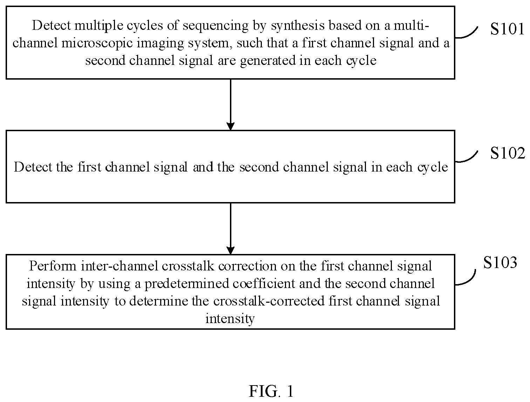

Referring to FIG. 1, a schematic flow chart of a method for determining inter-channel crosstalk correction strength provided in an embodiment of the present application is shown, and the method may include the following steps:

S101, detecting multiple cycles of sequencing by synthesis based on a multi-channel microscopic imaging system, such that a first channel signal and a second channel signal are generated in each cycle; and
detecting and obtaining sequencing information of the multiple cycles of sequencing by synthesis by using the multi-channel microscopic imaging system, wherein the first channel signal is indicative of a signal of incorporation of a first nucleotide into a plurality of identical polynucleotide molecules in the cycle, the second channel signal is indicative of a signal of incorporation of a second nucleotide into the plurality of identical polynucleotide molecules in the cycle, the first nucleotide and the second nucleotide are nucleotides comprising different types of bases, and a part of the first channel signal is noise related to second channel crosstalk; for example, for a sequencing platform that uses fluorescent molecules labeled differently to identify different bases, it may be detected that a fluorescence signal diffuses from one channel into another channel in one cycle of sequencing if the emission spectra of two or more selected fluorophores overlap;

S 102, detecting the first channel signal and the second channel signal in each cycle;

the first channel signal and the second channel signal both having intensity, wherein the intensity may characterize light intensity, and light intensity data may include pixel-related information, such as pixel grayscale values, of the first channel signal and the second channel signal on the base image; and

S103, performing inter-channel crosstalk correction on the first channel signal intensity by using a predetermined coefficient and the second channel signal intensity to determine the crosstalk-corrected first channel signal intensity; the predetermined coefficient being a fixed parameter associated with the multi-channel microscopic imaging system, i.e., a fixed parameter determined based on configuration information of the multi-channel microscopic imaging system.

In the embodiment of the present application, performing the inter-channel crosstalk correction on the first channel signal intensity by using the predetermined coefficient and the second channel signal intensity to determine the crosstalk-corrected first channel signal intensity includes:

determining the crosstalk-corrected first channel signal intensity by $I_1' = I_i - K \times I_2$,

wherein $I_1'$ is the crosstalk-corrected first channel signal intensity, $I_i$ is the detected first channel signal intensity, $I_2$ is the detected second channel signal intensity, and K is the predetermined coefficient.

When the first channel signal and the second channel signal are both fluorescence signals, determining the predetermined coefficient includes:

determining a range of possible values for the predetermined coefficient based on a spectral overlap region of a first channel and a second channel; and

determining a value that can meet a preset correction requirement to be the predetermined coefficient within the range of possible values.

In this embodiment, the range of possible values of the predetermined coefficient is determined based on spectra corresponding to the first channel signal and the second channel signal, and then the final predetermined coefficient is determined by traversing parameters.

Further, the process of determining a value that can meet a preset correction requirement to be the predetermined coefficient within the range of possible values may include:

determining the preset correction requirement based on the configuration information of the multi-channel microscopic imaging system; and

traversing corresponding values within the range of possible values according to traversal parameters, and determining the value that can meet the preset correction requirement to be the predetermined coefficient, wherein the traversal parameters include at least a step size parameter, and the step size parameter characterizes an interval parameter of two values in the traversal process.

When the preset correction requirement is determined based on the configuration information of the multi-channel microscopic imaging system, the preset correction requirement is mainly related to imaging configuration information of the multi-channel microscopic imaging system, such as hardware configuration information of the multi-channel microscopic imaging system, or optically detectable labels when the imaging is performed, and the like. Correspondingly, the preset correction requirement includes an alignment rate and/or an error rate. The alignment rate characterizes a ratio of the number of reads aligned to a reference genome to the total number of reads, i.e., alignment rate = the number of reads aligned to the reference genome/the total number of reads $\times$ 100%, wherein "the number of reads aligned to the reference genome" refers to the number of reads generated by sequencing that match the reference genome, and "the total number of reads" refers to the number of all reads generated by sequencing; the error rate characterizes a proportion of the number of bases that do not match the reference genome when reads generated by sequencing are aligned with the reference genome.

In the process of determining the predetermined coefficient, the determination may be made by target sample data with known base calling results. The base image obtained by the multi-channel microscopic imaging system may contain a plurality of signal points, and the optically detectable label may be a fluorophore, that is, a fluorescence signal emitted by a base carrying a fluorophore under the irradiation of a laser beam with a mixed wavelength may be acquired, and the acquired fluorescence signal is imaged by the multi-channel microscopic imaging system to obtain the base image. The plurality of signal points may be obtained from the base image, and relevant data such as the position and light intensity of each signal point may be obtained. The position may be a coordinate parameter, and the light intensity data may include pixel-related information, such as pixel grayscale values, of the signal point on the base image. When a base type corresponding to a certain signal point is determined, because a signal point in the base image corresponding to each type of base is obtained by exciting a label (such as a fluorophore) on the base by laser to generate a fluorescence signal, and then acquiring and imaging the fluorescence signal by the microscopic imaging system, the fluorophore on each type of base is excited to emit fluorescence with one wavelength, each channel signal is acquired and imaged by the microscopic imaging system, and if fluorescence spectra emitted by two or more fluorophores overlap, the inter-channel crosstalk occurs, causing the appearance of other base signals on the base image of a certain base, that is, a part of the first channel signal in the embodiment of the present application is noise related to second channel crosstalk, and thus, correct identification of the base cannot be performed. Therefore, it is necessary to perform crosstalk correction processing on the light intensity data of each channel signal, that is, after obtaining the predetermined coefficient, crosstalk correction may be performed on the first channel signal based on a general crosstalk correction method to determine the crosstalk-corrected first channel signal intensity.

When the predetermined coefficient for the crosstalk correction is determined based on the target sample data, the target sample data are light intensity data of the first channel signal and the second channel signal generated on the target sample, and then a crosstalk plot may be constructed by using the light intensity data. Each point in the crosstalk plot may represent one signal generated on the target sample, the abscissa in the crosstalk plot represents the light intensity of the first channel signal, and the ordinate represents the light intensity of the second channel signal. Two arms (or called arms) consisting of a plurality of points are shown in the figures, with an included angle between the arms. Repeated verification shows that when the crosstalk correction meets the preset correction requirement, i.e., the crosstalk correction meets the preset alignment rate and/or error rate, the included angle between the arms is a target angle value corresponding to 90 degrees, and the optimal condition is 90 degrees. In order to meet the preset correction requirement, corresponding values within the range of possible values are traversed according to the traversal parameters, and a value that can meet the preset correction requirement is determined to be the predetermined coefficient.

Specifically, when the included angle between the arms is 90 degrees, the most accurate predetermined coefficient can be obtained, but in practical applications, it has been found in studies that when the predetermined coefficient obtained in the case that the included angle between the arms fluctuates within a relatively small range of 90 degrees is applied, relatively accurate crosstalk correction results can also be obtained. Therefore, the preset correction requirement in the embodiment of the present application includes the included angle between the arms being a target angle value corresponding to 90 degrees, the target angle value including 90 degrees, and may also include a value within a relatively small range of upper and lower amplitudes corresponding to 90 degrees. The range may be determined according to practical application requirements, for example, the range may be within a range of plus or minus 1 degree or 0.5 degree of 90 degrees, and specifically, the first target angle value may be within a range of [89°, 91°]. In the process of traversing, verification may be performed once after one value is acquired, but if the range is relatively large and the number of points needing verification is relatively large, in order to improve the traversing efficiency, a next value to be traversed may be selected by presetting a step size parameter, and the step size parameter characterizes an interval parameter of two values in the traversal process. Based on the crosstalk plot, the intensity value of the first channel signal and the intensity value of the second channel signal corresponding to each point on the crosstalk plot are determined, the range of possible values is determined based on the intensity value of the first channel signal and the intensity value of the second channel signal of each point, and then further traversing is performed within this range to determine the predetermined parameter.

In this embodiment, the first channel may correspond to a signal channel of a first base, and the second channel may correspond to a signal channel of a second base, and in the crosstalk plot, each point may include signal intensity information of two types of bases, such as A and C bases, or A and G bases, respectively. The intensity value of the base corresponding to each point in the crosstalk plot may be a pixel grayscale value of the point on the base image. Based on the optimal value of a certain parameter to be found in the engineering debugging, a processing idea of a function evaluation method is generally adopted. That is, an index y capable of evaluating the influence of a parameter is found, the parameter x is traversed and sampled for verification to obtain a distribution curve or a partial distribution curve of a function $y = f(x)$, and a global optimal value or a local optimal value is found as a set value of the parameter x, thereby ensuring that the evaluation index y is in a relatively good interval. Two methods (i.e., two y indexes) for evaluating the crosstalk correction are provided corresponding to the embodiments of the present application, both of which may be used to determine the predetermined coefficient for the crosstalk correction.

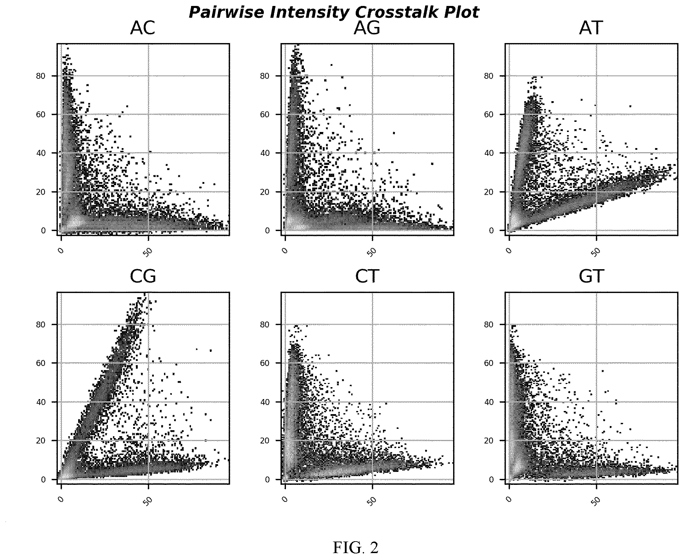

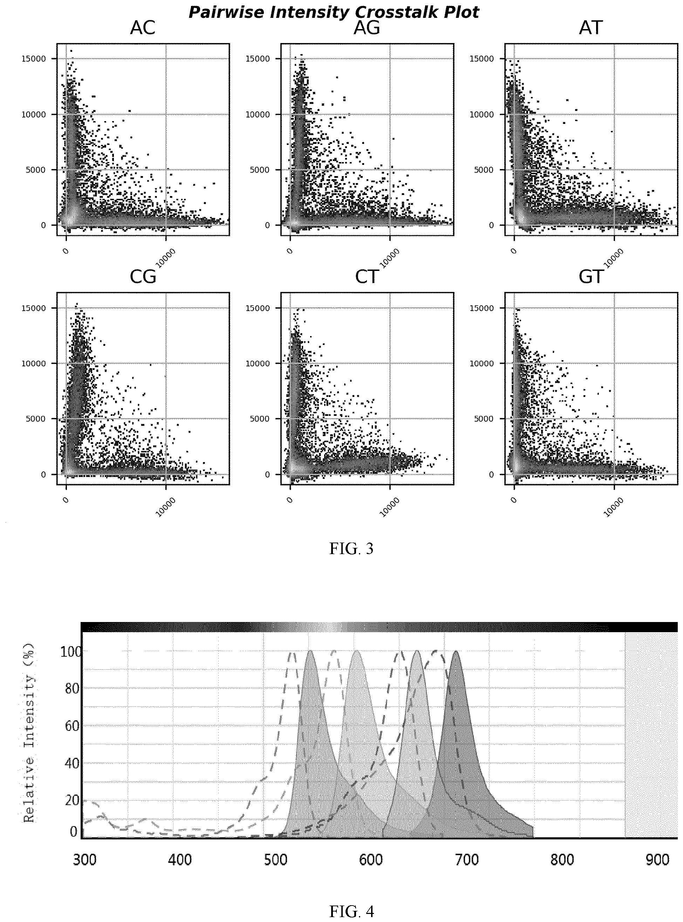

Method I is as described above, the crosstalk is from inter-channel signal energy crosstalk, and there are six combinations in total among the four types of bases of ACGT, see FIG. 2. FIG. 2 shows crosstalk plots. For example, there is relatively significant energy crosstalk between AT and CG, i.e., the included angle between the arms is an acute angle; there is no crosstalk between AC, i.e., the included angle between the corresponding arms is 90 degrees. It can be seen that after the crosstalk correction, it should be ensured that there is no crosstalk between any base combination, i.e., the effect that the included angle between the signal arms is 90 degrees (the arms are parallel to the respective axes) is achieved, such as the crosstalk plot corresponding to the AC bases in FIG. 2. Specifically, as can be seen from the crosstalk plots after the crosstalk correction shown in FIG. 3, the included angles between the arms all reach 90 degrees after the crosstalk correction, and the included angles in some plots after the correction do not reach 90 degrees, but are also similar to 90 degrees. In summary, whether the included angle between any two arms reaches 90 degrees and is parallel to the coordinate axis may be used as an evaluation index of the crosstalk correction effect.

Further, in order to reduce the number of traversals and reduce the occupation of computing resources, a specific range may be determined based on the intensity values corresponding to the bases represented by the abscissa and the ordinate in the current crosstalk plot, and then a specific step size parameter of traversal may be determined according to the specific range. For example, if the specific range is relatively large, the corresponding specific parameter step size is also relatively large, and if the specific range is relatively small, the corresponding specific parameter step size should also be relatively small in order to improve the accuracy. For example, all correction values between 0.01 and 1 may be traversed in a step size of 0.01. For example, when the crosstalk correction is performed on the AT crosstalk plot, see FIG. 3, the optimal value is reached when the AT correction value is 0.27, that is, the arm corresponding to the A base on the AT plot is parallel to the horizontal axis.

In the embodiment of the present application, the value that can meet the preset correction requirement is determined to be the predetermined coefficient, and the preset correction requirement includes an alignment rate and/or an error rate, wherein the alignment rate characterizes a ratio of the number of reads aligned to the reference genome to the total number of reads; the error rate characterizes a proportion of the number of bases that do not match the reference genome when reads generated by sequencing are aligned with the reference genome. Since the crosstalk correction is intended to obtain a more accurate base calling result, which is a part of the correction of grayscale values in the base image, the quality of the crosstalk correction may be determined by the key indexes of base calling, i.e., the alignment rate and the error rate. Specifically, a DNA insert of a known species (such as *Escherichia coli*) or a known specific tag sequence (such as a barcode/index sequence) is identified, and then the accuracy of the DNA insert or the accuracy of the tag sequence is determined by alignment to obtain a corresponding alignment rate and/or error rate index, thereby determining the quality of the predetermined coefficient. Under the condition that other algorithm modules are not changed, the closer the predetermined coefficient is to the real situation, the more reliable the identification result is, and the better the alignment index is.

Further, in order to quickly obtain a real and reliable predetermined coefficient for crosstalk correction, known spectral information and optical path design information of the multi-channel microscopic imaging system may also be used to calculate an inter-channel crosstalk ratio, thereby determining the predetermined coefficient.

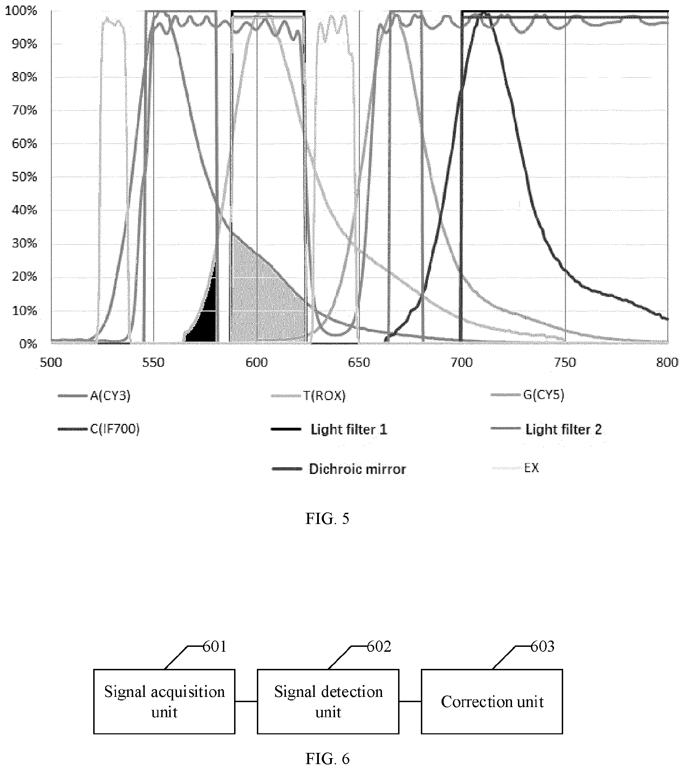

Specifically, as shown in FIGs. 4 and 5, FIG. 4 provides excitation spectra and emission spectra of different fluorophores, and FIG. 5 provides a schematic diagram of spectra of emission light from different fluorophores entering the microscopic imaging system. In the schematic diagram of spectra of FIG. 4, the dashed line represents the excitation spectrum, i.e., emitted by an exciter; the solid line represents the emission spectrum, i.e., emitted by different fluorophores (e.g., CY3, ROX, CY5, and IF700). In the schematic diagram of spectra of FIG. 5, four types of bases ATCG are labeled with different fluorophores, e.g., the A base labeled with CY3, the T base labeled with ROX, the G base labeled with CY5, and the C base labeled with IF700. As can be seen from FIG. 5, there is an overlap between the emission spectra of different fluorophores, for example, there is a partial overlap between the emission spectra of CY3 and ROX, and when the first channel signal and the second channel signal generated by CY3 and ROX, respectively, are detected by using the microscopic imaging system, the first channel signal may cause crosstalk into the second channel, and/or the second channel signal causes crosstalk into the first channel, that is, inter-channel crosstalk occurs. Although a dichroic mirror and a filter are arranged in the microscopic imaging system to split and filter the fluorescence signals generated by the two fluorophores (for example, filter 2 is used to transmit the fluorescence signal generated by ROX and filter the fluorescence signal generated by CY3; and filter 1 is used to transmit the fluorescence signal generated by CY3 and filter the fluorescence signal generated by ROX), the inter-channel crosstalk still occurs, which may be related to the optical path design of the microscopic imaging system. Therefore, known spectral information and optical path design information of the multi-channel microscopic imaging system may be used to theoretically calculate the energy crosstalk ratio between related channels, for example, as shown by the gray shaded portion and the black shaded portion shown in FIG. 5, so as to provide a theoretical reference value for the predetermined coefficient; moreover, fine adjustment may be performed near the theoretical value in the practical application process, the fine adjustment results take the highest alignment rate and the lowest error rate as the standards, and correspondingly, only one of the indexes can be taken as the correction target.

The present application adopts various methods to determine the predetermined coefficient, and corrects the inter-channel crosstalk by using the predetermined coefficient, thereby improving the accuracy and efficiency of the signal correction and/or base calling method.

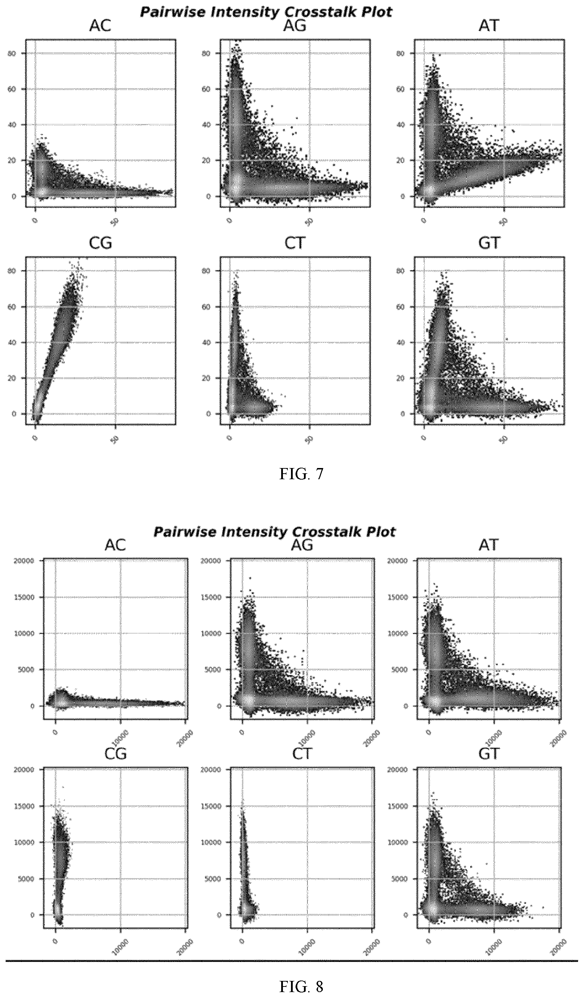

It should be noted that, in order to enable the determined predetermined coefficient for crosstalk correction to be applicable to more scenarios requiring crosstalk correction, the present application may also use special data to verify the preliminarily obtained predetermined coefficient, and if the verification is passed, the predetermined coefficient is determined, otherwise, the corresponding preliminarily obtained predetermined coefficient may be subjected to fine adjustment to obtain the predetermined coefficient. The phase-corrected value is significantly reduced in a relatively high sequencing cycle, and the phase-corrected value is substantially not changed in a relatively low sequencing cycle (the two types of crosstalk do not interfere with each other significantly). In order to be applicable to different numbers of sequencing cycles and corresponding scenarios, the embodiment of the present application verifies the determined fixed predetermined coefficient by using verification sample data with specific features, and the specific features include at least the existence of missing arms in the crosstalk plot. As shown in FIGs. 7 and 8, the embodiment of the present application verifies the determined fixed predetermined coefficient by using the verification sample data with missing arms in the crosstalk plot. The verification is passed, which in turn may also indicate that the predetermined coefficient for the crosstalk correction is applicable in the crosstalk correction scenario with missing arms. Further, the verification sample data with specific features for verification may include, for example, sequencing cycles with specific features. The sequencing cycles with specific features may be, for example, a specific number of sequencing cycles, and the specific number of sequencing cycles may represent a relatively high sequencing cycle or a relatively low sequencing cycle, which may be, for example, the 100th sequencing cycle or the 10th sequencing cycle.

After the signal crosstalk correction is performed by the predetermined coefficient, since there is also phase crosstalk between different sequencing cycles, phase correction may also be performed after the crosstalk correction is performed by the predetermined coefficient in the embodiment of the present application, that is, the fixed crosstalk correction is performed, and then the phase correction is performed.

In the embodiment of the present application, after performing the inter-channel crosstalk correction on the first channel signal intensity by using the predetermined coefficient and the second channel signal intensity, the method further includes:

performing phase correction on the first channel signal intensity after the inter-channel crosstalk correction by using the detected first channel signal intensity in the previous cycle and/or the next cycle to obtain the phase-corrected first channel signal intensity.

After the inter-channel crosstalk correction is performed, the first nucleotide incorporated into the plurality of identical polynucleotide molecules in the cycle may also be identified according to the crosstalk-corrected first channel signal intensity, thereby achieving base calling by the signal data after the phase correction.

Further, identifying the first nucleotide incorporated into the plurality of identical polynucleotide molecules in the cycle according to the phase-corrected first channel signal intensity includes: determining a normalization parameter based on the signal intensity of each channel; normalizing the phase-corrected first channel signal intensity based on the normalization parameter to obtain the normalized first channel signal intensity; and identifying the first nucleotide incorporated into the plurality of identical polynucleotide molecules in the cycle according to the normalized first channel signal intensity.

Since the crosstalk correction requires fixed parameters, when the parameters are fixed, it is found that there is a difference in the signal intensity due to the differences in excitation light power between different instruments and differences in devices used to carry test samples or differences in test samples. After the crosstalk correction is fixed, the intensity of a certain channel signal may be particularly large, which is not conducive to calculating the final corrected channel signal intensity. Therefore, it is necessary to normalize the intensity of each channel signal after the phase correction. The specific process is shown in the following formula:

$$newInts = K' \times (rawInts - pMvalue(AllTempBase))/(pNvalue(AllTempBase) - pMvalue(AllTempBase)),$$

wherein rawInts represents the signal intensity before normalization, newInts is the signal intensity after normalization, K' is a constant, pMvalue(AllTempBase) represents the channel signal intensity at the Mth quantile, pNvalue(AllTempBase) represents the channel signal intensity at the Nth quantile, and $N > M > 0$.

The above normalization can unify the final corrected channel signal intensity to a preset interval range for comparison.

It should be noted that in the embodiment of the present application, in order to achieve a better processing effect, a normalization process may also be performed before the crosstalk correction. In this normalization process, a zeroing without stretching processing method may be used to ensure that the background values of the channels are identical. In one embodiment, the normalization may be based on a template point of the image center, such as $800 \times 800$, and the translation and zeroing are performed. The following operations are independently performed on each channel:

$$newInts1 = rawInts1 - plvalue(midTemp),$$

wherein this process represents the normalization process before the crosstalk correction, newInts 1 represents the normalized channel signal intensity in this process, rawInts1 represents the channel signal intensity before normalization in this process, and p1value(midTemp) represents the channel signal intensity at the 1st quantile.

In the present application, phase correction is performed after crosstalk correction and supplemented with a normalization operation to complete base calling, further improving the accuracy of base calling.

In another embodiment of the present application, further provided is an apparatus for determining inter-channel crosstalk correction strength, see FIG. 6. The apparatus may comprise:

a signal acquisition unit 601, configured for detecting multiple cycles of sequencing by synthesis based on a multi-channel microscopic imaging system, such that a first channel signal and a second channel signal are generated in each cycle, wherein the first channel signal is indicative of a signal of incorporation of a first nucleotide into a plurality of identical polynucleotide molecules in the cycle, the second channel signal is indicative of a signal of incorporation of a second nucleotide into the plurality of identical polynucleotide molecules in the cycle, the first nucleotide and the second nucleotide are nucleotides comprising different types of bases, and a part of the first channel signal is noise related to second channel crosstalk;

a signal detection unit 602, configured for detecting the first channel signal and the second channel signal in each cycle, wherein the first channel signal and the second channel signal both have intensity; and

a correction unit 603, configured for performing inter-channel crosstalk correction on the first channel signal intensity by using a predetermined coefficient and the second channel signal intensity to determine the crosstalk-corrected first channel signal intensity, wherein the predetermined coefficient is a fixed coefficient associated with the multi-channel microscopic imaging system.

Optionally, the correction unit 603 comprises:

a first determining subunit, configured for determining the crosstalk-corrected first channel signal intensity by $I_1{}' = Ii - K \times I_2$,

wherein $I_1{}'$ is the crosstalk-corrected first channel signal intensity, Ii is the detected first channel signal intensity, $I_2$ is the detected second channel signal intensity, and K is the predetermined coefficient.

Optionally, the correction unit 603 comprises:

a phase correction subunit, configured for performing phase correction on the first channel signal intensity after the inter-channel crosstalk correction by using the detected first channel signal intensity in the previous cycle and/or the next cycle to obtain the phase-corrected first channel signal intensity.

Optionally, the apparatus further comprises:

an identification unit, configured for identifying the first nucleotide incorporated into the plurality of identical polynucleotide molecules in the cycle according to the phase-corrected first channel signal intensity.

Optionally, the apparatus further comprises a coefficient determining unit configured for determining the predetermined coefficient when the first channel signal and the second channel signal are both fluorescence signals. The coefficient determining unit comprises:

a second determining subunit, configured for determining a range of possible values for the predetermined coefficient based on a spectral overlap region of a first channel and a second channel; and

a third determining subunit, configured for determining a value that can meet a preset correction requirement as the predetermined coefficient within the range of possible values.

Optionally, the third determining subunit is specifically configured for:

determining the preset correction requirement based on the configuration information of the multi-channel microscopic imaging system; and

traversing corresponding values within the range of possible values according to traversal parameters, and determining the value that can meet the preset correction requirement as the predetermined coefficient, wherein the traversal parameters include at least a step size parameter, and the step size parameter characterizes an interval parameter of two values in the traversal process.

Optionally, the preset correction requirement includes an alignment rate and/or an error rate, wherein the alignment rate characterizes a ratio of the number of reads aligned to a reference genome to the total number of reads; the error rate characterizes a proportion of the number of bases that do not match the reference genome when reads generated by sequencing are aligned with the reference genome.

Optionally, the identification unit comprises:

a fourth determining subunit, configured for determining a normalization parameter based on the signal intensity of each channel;

a normalization subunit, configured for normalizing the corrected first channel signal intensity based on the normalization parameter to obtain the normalized first channel signal intensity; and

an identification subunit, configured for identifying the first nucleotide incorporated into the plurality of identical polynucleotide molecules in the cycle according to the normalized first channel signal intensity.

It should be noted that, in the present embodiment, reference may be made to the corresponding contents in the foregoing for specific implementations of each unit and subunit, and details are not described here.

In another embodiment of the present application, further provided is a computer-readable storage medium configured for storing a program executed by a computer, and executing the program includes implementing the method for determining the inter-channel crosstalk correction strength according to any one of the above.

In another embodiment of the present application, further provided is an electronic device, which may comprise:

a memory, configured for storing an application program and data generated by the operation of the application program; and

a processor, configured for executing the application program to implement the method for determining the inter-channel crosstalk correction strength according to any one of the above. It should be noted that, in the present embodiment, reference may be made to the corresponding contents in the foregoing for a specific implementation of the processor, and details are not described here.

The embodiments in this specification are all described in a progressive manner, each embodiment focuses on differences from other embodiments, and reference may be made to each other for identical and similar parts between the embodiments. Since the apparatus disclosed in the embodiment corresponds to the method disclosed in the embodiment, the description is relatively simple, and reference may be made to the partial description of the method.

Those skilled in the art may further appreciate that the units and algorithm steps of various embodiments described in connection with the embodiments disclosed herein may be implemented in electronic hardware, computer software, or a combination thereof. In order to clearly illustrate the interchangeability of hardware and software, the components and steps of various embodiments have been generally described in the above description according to functions. Whether these functions are performed in hardware or software depends on the particular application and design constraints of the technical solutions. Those skilled in the art may implement the described functions in varying ways for each particular application, but such implementations should not be interpreted as departing from the scope of the present application.

The method or algorithm steps described in connection with the embodiments disclosed herein may be implemented directly with hardware, a software module executed by the processor, or a combination thereof. The software module may be placed in a random access memory (RAM), a memory, a read-only memory (ROM), an electrically programmable ROM, an electrically erasable programmable ROM, a register, a hard disk, a removable disk, a CD-ROM, or any other forms of storage media well known in the art.

The above description of the disclosed embodiments enables those skilled in the art to implement or use the present application. Various modifications to these embodiments will be apparent to those skilled in the art, and the generic principles defined herein may be implemented in other embodiments without departing from the spirit or scope of the present application. Thus, the present application is not limited to these embodiments shown herein, but accords with the broadest scope consistent with the principles and novel features disclosed herein.

## Claims

1. A method for determining inter-channel crosstalk correction strength, comprising:

detecting multiple cycles of sequencing by synthesis based on a multi-channel microscopic imaging system, such that a first channel signal and a second channel signal are generated in each cycle, wherein the first channel signal is indicative of a signal of incorporation of a first nucleotide into a plurality of identical polynucleotide molecules in the cycle, the second channel signal is indicative of a signal of incorporation of a second nucleotide into the plurality of identical polynucleotide molecules in the cycle, the first nucleotide and the second nucleotide are nucleotides comprising different types of bases, and a part of the first channel signal is noise related to second channel crosstalk;

detecting the first channel signal and the second channel signal in each cycle, wherein the first channel signal and the second channel signal both have intensity;

and performing inter-channel crosstalk correction on the first channel signal intensity by using a predetermined coefficient and the second channel signal intensity to determine the crosstalk-corrected first channel signal intensity, wherein the predetermined coefficient is a fixed coefficient associated with the multi-channel microscopic imaging system.

2. The method according to claim 1, wherein performing the inter-channel crosstalk correction on the first channel signal intensity by using the predetermined coefficient and the second channel signal intensity to determine the crosstalk-corrected first channel signal intensity comprises:

determining the crosstalk-corrected first channel signal intensity by $I_1{'} = Ii - K \times I_2$,
wherein $I_1{'}$ is the crosstalk-corrected first channel signal intensity, $Ii$ is the detected first channel signal intensity, $I_2$ is the detected second channel signal intensity, and K is the predetermined coefficient.

3. The method according to claim 1 or 2, further comprising, after performing the inter-channel crosstalk correction on the first channel signal intensity by using the predetermined coefficient and the second channel signal intensity:
performing phase correction on the first channel signal intensity after the inter-channel crosstalk correction by using the detected first channel signal intensity in the previous cycle and/or the next cycle to obtain the phase-corrected first channel signal intensity.

4. The method according to claim 3, further comprising:
identifying the first nucleotide incorporated into the plurality of identical polynucleotide molecules in the cycle according to the phase-corrected first channel signal intensity.

5. The method according to claim 4, wherein identifying the first nucleotide incorporated into the plurality of identical polynucleotide molecules in the cycle according to the phase-corrected first channel signal intensity comprises:

determining a normalization parameter based on the signal intensity of each channel;
normalizing the phase-corrected first channel signal intensity based on the normalization parameter to obtain the normalized first channel signal intensity; and
identifying the first nucleotide incorporated into the plurality of identical polynucleotide molecules in the cycle according to the normalized first channel signal intensity.

6. The method according to any one of claims 1-5, wherein the first channel signal and the second channel signal are both fluorescence signals, and determining the predetermined coefficient comprises:

determining a range of possible values for the predetermined coefficient based on a spectral overlap region of a first channel and a second channel; and
determining a value that can meet a preset correction requirement as the predetermined coefficient within the range of possible values.

7. The method according to claim 6, wherein the determining the value that can meet the preset correction requirement as the predetermined coefficient within the range of possible values comprises:

determining the preset correction requirement based on configuration information of the multi-channel microscopic imaging system; and
traversing corresponding values within the range of possible values according to traversal parameters, and determining the value that can meet the preset correction requirement as the predetermined coefficient, wherein the traversal parameters comprise at least a step size parameter, and the step size parameter characterizes an interval parameter of two values in the traversal process.

8. The method according to claim 7, wherein the preset correction requirement comprises an alignment rate and/or an error rate, wherein the alignment rate characterizes a ratio of the number of reads aligned to a reference genome to the total number of reads;
the error rate characterizes a proportion of the number of bases that do not match the reference genome when reads generated by sequencing are aligned with the reference genome.

**9.** An apparatus for determining inter-channel crosstalk correction strength, comprising:

a signal acquisition unit, configured for detecting multiple cycles of sequencing by synthesis based on a multi-channel microscopic imaging system, such that a first channel signal and a second channel signal are generated in each cycle, wherein the first channel signal is indicative of a signal of incorporation of a first nucleotide into a plurality of identical polynucleotide molecules in the cycle, the second channel signal is indicative of a signal of incorporation of a second nucleotide into the plurality of identical polynucleotide molecules in the cycle, the first nucleotide and the second nucleotide are nucleotides comprising different types of bases, and a part of the first channel signal is noise related to second channel crosstalk;

a signal detection unit, configured for detecting the first channel signal and the second channel signal in each cycle, wherein the first channel signal and the second channel signal both have intensity; and

a correction unit, configured for performing inter-channel crosstalk correction on the first channel signal intensity by using a predetermined coefficient and the second channel signal intensity to determine the crosstalk-corrected first channel signal intensity, wherein the predetermined coefficient is a fixed coefficient associated with the multi-channel microscopic imaging system.

**10.** The apparatus according to claim 9, wherein the correction unit comprises:

a first determining subunit, configured for determining the crosstalk-corrected first channel signal intensity by $I_1' = Ii - K \times I_2$,

wherein $I_1'$ is the crosstalk-corrected first channel signal intensity, Ii is the detected first channel signal intensity, $I_2$ is the detected second channel signal intensity, and K is the predetermined coefficient.

**11.** The apparatus according to claim 9 or 10, wherein the correction unit comprises:

a phase correction subunit, configured for performing phase correction on the first channel signal intensity after the inter-channel crosstalk correction by using the detected first channel signal intensity in the previous cycle and/or the next cycle to obtain the phase-corrected first channel signal intensity.

**12.** The apparatus according to claim 11, further comprising:

an identification unit, configured for identifying the first nucleotide incorporated into the plurality of identical polynucleotide molecules in the cycle according to the phase-corrected first channel signal intensity;

wherein the identification unit optionally comprises:

a fourth determining subunit, configured for determining a normalization parameter based on the signal intensity of each channel;

a normalization subunit, configured for normalizing the phase-corrected first channel signal intensity based on the normalization parameter to obtain the normalized first channel signal intensity; and

an identification subunit, configured for identifying the first nucleotide incorporated into the plurality of identical polynucleotide molecules in the cycle according to the normalized first channel signal intensity.

**13.** The apparatus according to any one of claims 9-12, further comprising a coefficient determining unit configured for determining the predetermined coefficient when the first channel signal and the second channel signal are both fluorescence signals, wherein the coefficient determining unit comprises:

a second determining subunit, configured for determining a range of possible values for the predetermined coefficient based on a spectral overlap region of a first channel and a second channel; and

a third determining subunit, configured for determining a value that can meet a preset correction requirement as the predetermined coefficient within the range of possible values.

**14.** The apparatus according to claim 13, wherein the third determining subunit is specifically configured for:

determining the preset correction requirement based on configuration information of the multi-channel microscopic imaging system; and

traversing corresponding values within the range of possible values according to traversal parameters, and determining the value that can meet the preset correction requirement as the predetermined coefficient, wherein the traversal parameters comprise at least a step size parameter, and the step size parameter characterizes an interval parameter of two values in the traversal process;

wherein optionally the preset correction requirement comprises an alignment rate and/or an error rate, wherein the alignment rate characterizes a ratio of the number of reads aligned to a reference genome to the total number of reads;

the error rate characterizes a proportion of the number of bases that do not match the reference genome when reads generated by sequencing are aligned with the reference genome.

15. A computer-readable storage medium having a program stored thereon, wherein the program can be executed by a processor to implement the method for determining the inter-channel crosstalk correction strength according to any one of claims 1-8.

Detect multiple cycles of sequencing by synthesis based on a multi-channel microscopic imaging system, such that a first channel signal and a second channel signal are generated in each cycle — S101

Detect the first channel signal and the second channel signal in each cycle — S102

Perform inter-channel crosstalk correction on the first channel signal intensity by using a predetermined coefficient and the second channel signal intensity to determine the crosstalk-corrected first channel signal intensity — S103

FIG. 1

FIG. 2

## Pairwise Intensity Crosstalk Plot

FIG. 3

FIG. 4

FIG. 5

FIG. 6

FIG. 7

FIG. 8

Europäisches
Patentamt

European
Patent Office

Office européen
des brevets

# EUROPEAN SEARCH REPORT

**Application Number**

EP 24 22 0466

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/080142 A1 (LANGLOIS ROBERT [US] ET AL) 12 March 2020 (2020-03-12) * paragraphs [0015], [0040], [0050], [0076] - [0078] * | 1-15 | INV. G06V10/30 C12Q1/6874 G01N21/64 G06V20/69 |
| X | CN 115 035 952 A (SHENZHEN SAILU MEDICAL TECH CO LTD) 9 September 2022 (2022-09-09) * the whole document * & US 2025/080229 A1 (CHEN WEI [CN] ET AL) 6 March 2025 (2025-03-06) * paragraphs [0064] - [0093] * | 1,9,15 | |
| X | US 2023/351598 A1 (GARCIA FRANCISCO JOSE [US] ET AL) 2 November 2023 (2023-11-02) * paragraphs [0059] - [0072] * | 1,9,15 | |

**TECHNICAL FIELDS SEARCHED (IPC)**

C12Q
G01N
G06V

The present search report has been drawn up for all claims

| Place of search | Date of completion of the search | Examiner |
|---|---|---|
| The Hague | 12 May 2025 | Mukasa, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 22 0466

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2025

| Patent document cited in search report | | Publication date | Patent family member(s) | | Publication date |
|---|---|---|---|---|---|
| US 2020080142 | A1 | 12-03-2020 | AU | 2018231017 A1 | 20-06-2019 |
| | | | AU | 2021269291 A1 | 09-12-2021 |
| | | | BR | 112019014683 A2 | 18-02-2020 |
| | | | CA | 3046015 A1 | 13-09-2018 |
| | | | CN | 110234771 A | 13-09-2019 |
| | | | EP | 3592865 A1 | 15-01-2020 |
| | | | IL | 267051 A | 29-08-2019 |
| | | | JP | 7041684 B2 | 24-03-2022 |
| | | | JP | 7655879 B2 | 02-04-2025 |
| | | | JP | 2020507758 A | 12-03-2020 |
| | | | JP | 2022088444 A | 14-06-2022 |
| | | | JP | 2024028778 A | 05-03-2024 |
| | | | KR | 20190103267 A | 04-09-2019 |
| | | | KR | 20210047980 A | 30-04-2021 |
| | | | NZ | 754255 A | 28-05-2021 |
| | | | RU | 2736384 C1 | 16-11-2020 |
| | | | US | 2020080142 A1 | 12-03-2020 |
| | | | WO | 2018165099 A1 | 13-09-2018 |
| | | | ZA | 202106656 B | 27-07-2022 |
| CN 115035952 | A | 09-09-2022 | CN | 115035952 A | 09-09-2022 |
| | | | EP | 4528742 A1 | 26-03-2025 |
| | | | US | 2025080229 A1 | 06-03-2025 |
| | | | WO | 2023221546 A1 | 23-11-2023 |
| US 2023351598 | A1 | 02-11-2023 | US | 2012020537 A1 | 26-01-2012 |
| | | | US | 2015243028 A1 | 27-08-2015 |
| | | | US | 2017161900 A1 | 08-06-2017 |
| | | | US | 2020074630 A1 | 05-03-2020 |
| | | | US | 2021150715 A1 | 20-05-2021 |
| | | | US | 2022051407 A1 | 17-02-2022 |
| | | | US | 2023351598 A1 | 02-11-2023 |
| | | | US | 2025148603 A1 | 08-05-2025 |
| | | | US | 2025148604 A1 | 08-05-2025 |

EPO FORM P0459

For more details about this annex : see Official Journal of the European Patent Office, No. 12/82